# EUROPEAN PATENT APPLICATION

(11) **EP 4 563 685 A1**
(43) Date of publication of application: **04.06.2025**
(21) Application number: 24216644.5
(22) Date of filing: 29.11.2024
(51) Int. Cl.: C12G 1/02, G01N 33/02, G05B 21/00, G05D 7/00, G05D 21/00, B01F 33/40

(54) **APPARATUS AND RELATED PROCESS FOR THE VINIFICATION OF GRAPE MUST**

(30) Priority: 30.11.2023 IT 202300025509
(71) Applicant: Gimar S.r.l., 15040 Occimiano (AL) (IT)
(72) Inventor: LAMBRI, Milena, PIACENZA (IT); FRANCIA, Marco, CASALE MONFERRATO (AT) (IT)
(74) Representative: Silva, Valentina

(57) **Abstract**

Apparatus (10) usable for the vinification of a liquid must with its lees, in particular for white wine making, both of white grapes and of red grapes, said apparatus (10) comprising a tank (22) for containing the must/wine, characterised in that said tank (22) comprises active means (24) on the must/wine, in particular comprising means (250) for introducing a technical gas operatively connected to at least first supply means (251) for introducing into the must/wine at least a first technical gas (G1) and to at least second supply means (252) for introducing into the must/wine at least a second technical gas (G2) of a different type, wherein said means (250) for introducing a gas comprise a distributor (250b) placed within the tank (22) and positioned substantially in the centre of the tank (22).

## Description

### FIELD OF APPLICATION OF THE INVENTION

The present invention relates to the field of oenological processes.

Specifically, it refers to an apparatus and related process for the vinification of grape must.

In particular, said grape must comprises liquid must with its lees.

In particular, reference will be made to white vinification, i.e. without marc, for the production of white and/or rose wines.

### STATE OF THE ART

Apparatuses and processes that can be used for the white vinification of grape must are known.

In particular, vinification apparatus generally includes a must holding tank.

In the tank, the main vinification steps take place, such as the settling, alcoholic fermentation and refinement on the lees.

The biological, chemical and physical processes involved in the transformation of must into wine are very complex, interacting with each other and dependent on the conditions in which they take place. Monitoring and prompt intervention during these processes has a strong impact on the quality of the final product.

Known tanks are equipped with systems for monitoring the must during said vinification steps.

For example, it is known to control the temperature of the must to ensure that the fermentation process runs smoothly.

In particular, the thermal conditioning of fermenting musts is normally performed with the use of forced circulation of cooling or heating solutions within heat exchangers installed on the tank that provide heat exchange with the fermenting product.

Thermal conditioning as described is widely used and, when present, is performed automatically.

Technological progress has obviously affected not only conditioning, but also various other aspects of these tanks, which are now often equipped with modern and sophisticated devices, often controlled by computers.

According to the known technique, however, only certain monitoring is carried out during the transformation steps, in particular for the white vinification of must into wine, and the vinification apparatus is not always equipped with all the necessary means to take rapid and controlled action in response to such monitoring.

A need is thus perceived in the sector for apparatus that allows the must to be vinified, particularly in white wine, with its lees in a more controlled and automated manner, and therefore faster and safer.

This is especially necessary with regard to the vinification of rosé and white wines, which are more delicate, as they are strongly affected by the conditions in which vinification takes place.

In this case, there is a particular need for apparatus to control all the vinification steps, such as settling, fermentation, refinement/aging, dosing appropriately and selectively, technical gases, necessary for the correct performance of these steps in order to raise the quality level of the product to be made.

A further purpose of the invention concerns the fact that it is possible to use an apparatus that allows the same must to be vinified to obtain wines with different sensory profiles by means of a different controlled input of technical gases.

A further purpose of the invention is to be able to use an apparatus with a tank for the long-term storage of wine, thus limiting degradation to a minimum.

There is also a need in the industry to optimally manage the vinification process, avoiding or reducing to a maximum degree the intervention of the operator or oenologist, in particular to reduce human errors and to increase the sustainability of vinification, minimising or cancelling corrective actions and increased costs that are necessary due to errors.

With the present invention it is therefore desirable to propose a new solution with respect to the solutions known up to now and in particular it is proposed to overcome one or more of the drawbacks or problems referred to above, and/or to satisfy one or more requirements referred to above and/or in any case experienced in the art, and in particular which can be deduced from the above.

In particular, the subject of the invention is an apparatus that can be used for the vinification of liquid must with its lees.

Preferably, the apparatus can be used for white vinification of both white and red grapes.

Preferably said apparatus comprises a must/wine containment tank.

In an advantageous form, said tank comprises active means on the must/wine, in particular including means for introducing a technical gas.

Advantageously, said means for introducing a technical gas are operatively connected to at least first supply means for introducing into the must/wine at least a first technical gas and to at least second supply means for introducing into the must/wine at least a second technical gas of a different type.

Preferably, said means for introducing a gas comprise a distributor placed inside the tank and positioned substantially in the centre of the tank.

Preferably the distributor is positioned at a lower portion of the tank.

Preferably the distributor is near a bottom wall of the tank.

Preferably the first technical gas is an inert gas and preferably comprises and/or consists of nitrogen and/or carbon dioxide.

Preferably the second technical gas is a non-inert gas and preferably comprises and/or consists of oxygen and/or air.

Preferably, the active means comprise activation means for placing in fluid connection said means for introducing a gas with the first supply means or with the second supply means.

Preferably, the activation means place in fluid connection, in an alternative manner, said means for introducing a gas with the first supply means or with the second supply means.

Preferably, the activation means are adapted to control the time and/or frequency of introducing the first gas and/or second gas.

Preferably said means for introducing a gas comprise a conduit in fluid connection with the distributor.

Preferably said conduit is installed at a lower portion of the tank in particular near the bottom wall of the tank.

Advantageously, said conduit is connected to the tank by entering from an outlet conduit of said tank.

Preferably the distributor comprises a plurality of holes particularly distributed on its outer surface.

Advantageously, the distributor is shaped like a ball or ring preferably having a diameter comprised between 25 and 50 mm.

Preferably the distributor, in particular the ball or ring comprises holes having a diameter comprised between 1 to 3 mm, preferably 1.5 mm.

Preferably the apparatus comprises means for detecting physical and/or chemical parameters of said must/wine.

Preferably it comprises means for detecting the temperature, especially by means of a corresponding sensor.

Preferably it comprises means for detecting the density of the must, especially by means of a corresponding sensor, i.e. a first and a second sensor.

Preferably it comprises means for detecting the redox potential, in particular by means of a corresponding sensor.

Preferably it comprises means for detecting the amount of oxygen dissolved in the must, in particular by means of a corresponding sensor.

Preferably it comprises means for detecting the colouring extracted during maceration, in particular by means of a corresponding sensor.

Preferably it comprises means for detecting the flow of liquid and/or gaseous fluids within the conduit, in particular by means of a corresponding flow sensor.

Preferably, the apparatus comprises processor means, input means of corresponding data and/or signals into said processor means, and output means of corresponding data and/or signals originating from said processor means.

Preferably said input means of corresponding data and/or signals into said processor means comprise a respective keyboard, or equivalent, for input of corresponding commands and/or said means of detecting physical and/or chemical parameters of said must/wine.

Preferably said output means of corresponding data and/or signals originating from said processor means comprise a display screen of said processor means and/or said means active on the must/wine.

Preferably said processor means are configured to activate, in particular automatically, one or more of said active means on the must/wine or in response to a signal received from said means for detecting physical and/or chemical parameters of said must/wine.

Preferably, said processor means use at least one vinification recipe or a collection or set of vinification recipes, housed in a corresponding memory of, or in connection with, said processor means.

The invention also relates to the process that can be used for the vinification of a must with its lees, in particular for white wine making, both of white grapes and of red grapes.

Said process uses a corresponding apparatus for vinification.

Preferably, said process involves introducing into the must/wine, during the vinification steps, at least a first technical gas in a respective vinification step and a second gas of a different type in a respective vinification step.

Preferably said introduction is made from the centre of the tank.

Preferably the process provides to perform vinification steps in the tank comprising an initial step of settling and/or an alcoholic fermentation step and/or a refining step.

Preferably the process provides by means of processor means of the apparatus to activate the active means in particular the first supply means and/or the second supply means in response to a signal received by said means for detecting physical and/or chemical parameters of said must/wine.

Preferably the process following a signal received from said sensor of the redox potential, provides by means of the processor means to activate the first power supply means, if the detected potential is lower than the predetermined minimum potential.

Preferably the process following a signal received from said sensor of the redox potential, provides by means of the processor means to lock the first power supply means, if the detected potential is greater than the predetermined maximum potential.

Preferably the process provides by means of said processor means of the apparatus to use at least one vinification recipe or a collection or set of vinification recipes, preferably housed in a corresponding memory of, or in connection with processor means, wherein said recipe provides to introduce the first gas and/or the second gas of different types into the tank during the execution of a respective vinification step.

Preferably, the process provides that the recipe defines the activation/deactivation of the active means and/or their activation/deactivation time and/or their activation/deactivation frequency of introduction of the first gas and the second gas for each vinification step.

This and other innovative aspects or respective advantageous embodiments, are however, set forth in the appended claims, the technical characteristics of which can be found, together with corresponding advantages achieved, in the following detailed description, illustrating a purely non-limiting example of the embodiment of the invention, which is made with reference to the appended drawings, in which:
- Figure 1a illustrates a front elevated schematic view of a preferred embodiment of the apparatus implementing the process according to the present invention;
- Figure 1b illustrates a front elevated schematic view of a preferred embodiment of the apparatus implementing the process according to the present invention;

- Figure 2a shows a schematic view of a detail of Figure 1a in particular of the active means;
- Figure 2b shows a schematic view of a detail of Figure 1b in particular of the active means;
- Figure 3a shows a schematic view of a distributor of the active means in Figure 1a;
- Figure 3b shows a schematic view of a distributor of the active means in Figure 1b;
- Figure 4 shows a schematic view of an interface of the processor means;
- Figures 5a, 5b, 5c show a view of a graphical user interface implemented by the embodiment of the vinification steps according to the present invention.

The accompanying figures show a preferred embodiment of the apparatus 10 that can be used for vinification, of a grape must, comprising liquid must with its lees, for white wine making, i.e. without marc.

The process of the invention uses the corresponding apparatus 10.

In particular, reference will be made to a process in which the vinification steps are more specifically defined to obtain rosé wines or white wines.

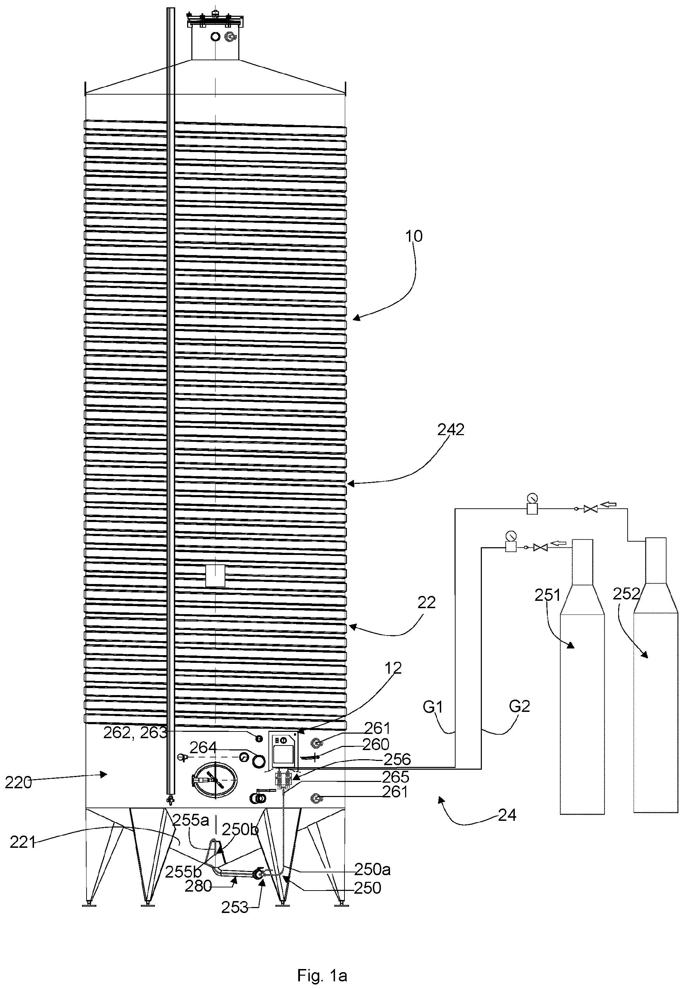

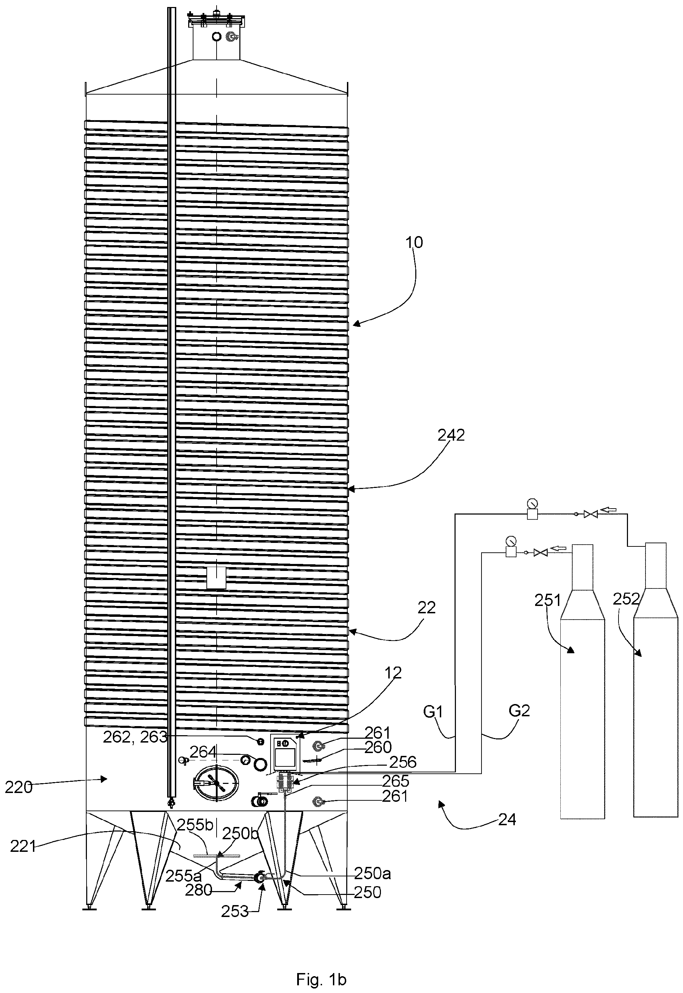

In particular, said apparatus 10 comprises a must holding tank 22, as depicted in Figures 1a, 1b.

Said tank 22 comprises a lower portion 220 delimited at the bottom by a bottom wall 221a defining a bottom 221 of the tank 22.

This bottom wall 221 can be made in different conformations.

It can be, for example, a flat, slightly inclined, or conical bottom wall 221a. The figures show an embodiment in which the tank comprises a conical 221a bottom wall.

It is understood, however, that this representation is not limiting and the subject matter of the invention may be applied to a tank 22 with a different conformation.

The must contained within the tank 22 is subjected to a vinification process involving the following steps in particular:
- a first step of must settling,
- a second step of alcoholic fermentation of the must
- a third step of refinement/aging on the wine lees.

According to an aspect of said invention, said tank 22 comprises active means 24 on the must/wine that act during the said vinification steps.

As can be seen from Figures 1a and 1b, said apparatus 10 comprises, for example, as active means 24 on the must/wine, one or more thermal conditioning means 242, in particular for cooling or heating said must/wine, preferably comprising a heat exchange coil surrounding said must/wine containment tank 22.

Generally, during said steps there is interaction with technical gases G1, G2 that must be blown into the tank 22 to ensure the correct progress of the vinification steps and to reduce the possibility of must/wine defects.

According to an advantageous embodiment of the invention, said active means 24, include means 250 for introducing technical gases G1, G2.

According to an aspect of the invention, said introducing means 250 are operatively connected to at least first supply means 251 for introducing into the must/wine at least a first gas G1 and are operatively connected to at least second supply means 252 for introducing into the must/wine at least a second technical gas G2 of a different type.

Indeed, during the vinification steps it may be necessary for the must/wine to come into contact with different types of technical gases G1, G2.

In particular, two types are identified: an inert gas G1 and a non-inert gas G2.

The inert gases G1 include e.g. nitrogen, argon, carbon dioxide, etc.

The non-inert gases G1 include e.g. oxygen, air, etc.

Depending on the vinification step underway and the values determined from physical/chemical checks on the must/wine, the type of gas G1, G2 to be introduced is defined.

For example, it may be necessary to insufflate an inert gas G1 in a first settling step.

In this case, insufflating said inert gas G1 into the must has the main purpose of putting the solid material, lees and clarification substances back into suspension.

This makes it possible to optimise the extraction of flavours, polysaccharides, organic nitrogen, etc., and to facilitate their dispersion and solubilisation in the must.

For example, in a second step of alcoholic fermentation, it may be necessary to insufflate a non-inert gas G2 containing oxygen to encourage better yeast viability and thus promote more regular fermentation kinetics, to assist the same yeasts in utilising the various forms of nitrogen naturally present in the must to modulate the formation of fermentation aroma with different composition in esters, aldehydes, alcohols, etc.

In said alcoholic fermentation step, it may also be necessary to insufflate an inert gas G1, if a reduction fermentation is to be carried out, modulating the formation of thiol aromas, e.g. for musts obtained from certain characterised grape varieties, or to increase the floral aroma component from other aroma precursors such as norisoprenoids and terpenes.

For example, in a third step of refining on the lees, it may be necessary to insufflate an inert gas G1 into the wine, the main purpose of which is to bring the solid material, i.e. the fermentation lees, back into suspension.

In said third step of refining on the lees, it may also be necessary to insufflate a non-inert gas G2 such as air or oxygen, generally in low quantities. This non-inert gas makes it possible to avoid a state of excessive lees reduction when highly unpleasant sulphides would be formed and, therefore, detrimental to the quality of the wine, as precursors of mercaptans that are difficult to remove.

Thanks to said means 250 for introducing technical gases G1, G2, it is possible to control the fermentation process, e.g. by dosing the amount of oxygen contained in the must, or by insufflating only inert gases.

According to an aspect of the invention, the first supply means 251 and/or second supply means 252 comprise a generator of the first or second technical gas G1, G2.

Alternatively, the first supply means 251 and/or second supply means 252 may comprise a cylinder of the first or second technical gas G1, G2.

With regard to the supply means 252 for supplying air, it is preferably taken from a compressed air source, in which there are filter units of a known type, adapted to cleanse and purify it appropriately.

Alternatively, compressed air is also supplied by means 252 such as a pressurised cylinder.

We will not go into more detail about said first and second supply means 251, 252 as they are known to a person skilled in the art.

However, it is understood that supply means capable of supplying an inert gas G1 and/or a non-inert gas G2, but of a different type from those mentioned above, are also within the scope of the invention.

According to a preferred embodiment depicted in the figures, said means 250 for introducing a gas comprise a conduit 250a installed at the lower portion 220 of the tank 22.

In particular, said conduit 250a is installed near the bottom wall 221a of the tank 22.

This allows technical gases G1, G2 to be insufflated directly in contact with the must/wine regardless of the level contained in the tank 22.

A preferred embodiment provides that said conduit 250a enters the tank 22 through the bottom wall 221a.

Preferably, said conduit 250a enters the tank 22 substantially in the centre of said bottom wall 221a. A preferred embodiment, depicted in the figure, envisages that said conduit 250a is connected to the tank 22 by entering from an outlet conduit 280 of said tank 22.

This makes it possible to make use of the outlet conduit 280 which is always present in vinification tanks 22, even in less well-equipped ones.

This simplifies in existing tanks 22 the step of installing said active means 24 in particular of said means 250 for injecting a gas.

According to an aspect of the invention said means 250 for introducing a gas comprise a distributor 250b in fluid connection with the conduit 250a.

Via the same distributor 250b, both the inert gas G1 and the non-inert gas G2 can be introduced into the tank 22.

This simplifies and optimises the solution, because it allows the two technical gases G1, G2 to be supplied alternatively with just one distributor 250b.

Said distributor 250b is preferably positioned inside the tank 22 and preferably at the lower portion 220 thereof.

In particular, said distributor 250b is positioned substantially in the centre of the tank 22 preferably near the bottom wall 221a.

This allows the first or second technical gas G1, G2 to be introduced from the centre outwards, a position that is particularly effective in suspending the lees.

In the event that the tank 22 has a conical bottom wall 221a, as shown in the figures, said distributor 250b can be positioned in the conical portion of the bottom 221.

In this case, the distributor 250b is preferably positioned at the axis of symmetry X of the cone defined by the bottom wall 221a.

Also preferably, the conduit 250a is positioned at the axis of the cone defined by the bottom wall 221a.

A possible embodiment, not shown in the figure, but particularly suitable for large-diameter tanks 22, may provide for a plurality of distributors 250b, preferably positioned inside the tank 22 and preferably at its lower portion 220, in particular positioned near the bottom wall 221a.

In particular, said distributors 250b are positioned substantially in the centre of the bottom wall 221a.

Thanks to this position, it is possible to resuspend most of the lees deposited on the bottom 221.

In the case of the tank 22 with only one distributor 250b, as shown in the figure, said distributor 250b is preferably positioned inside the tank 22 in a central position.

This allows for a particularly effective diffusion action of the gas bubbles so that they are evenly and homogeneously dispersed in the liquid.

Preferably said distributor 250b comprises a plurality of holes.

Said holes are in particular evenly distributed on the outer surface of said distributor 250b.

This allows a controlled and uniform distribution of the first or second gas G1, G2 throughout the must/wine.

Thanks to said distributor 250b, it is possible to carry out both a must/wine stirring action, with adequate re-suspension of the lees, and a micro-oxygenation action.

Preferably, the conduit 250a comprises a portion of conduit 255a which is positioned completely inside the tank 22.

Said portion of conduit 255a is mechanically connected to the distributor 250b.

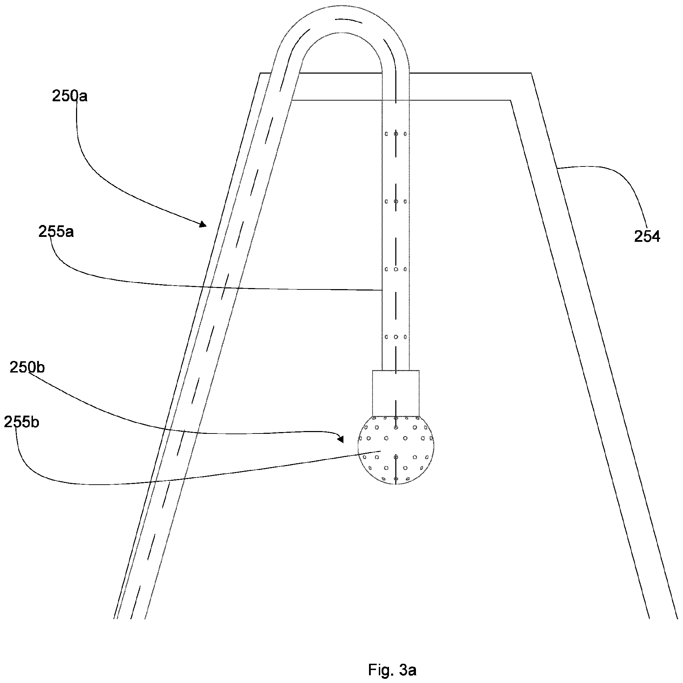

Said portion of conduit 255a may comprise a plurality of holes distributed over its surface as depicted in Figure 3a.

This allows with the introduction of the first or second technical gas G1, G2 to suspend more lees located in the lowest part of the bottom 221, i.e. on the bottom wall 221a.

Preferably this portion of the conduit 255a is positioned centrally to the bottom wall 221 of the tank 22.

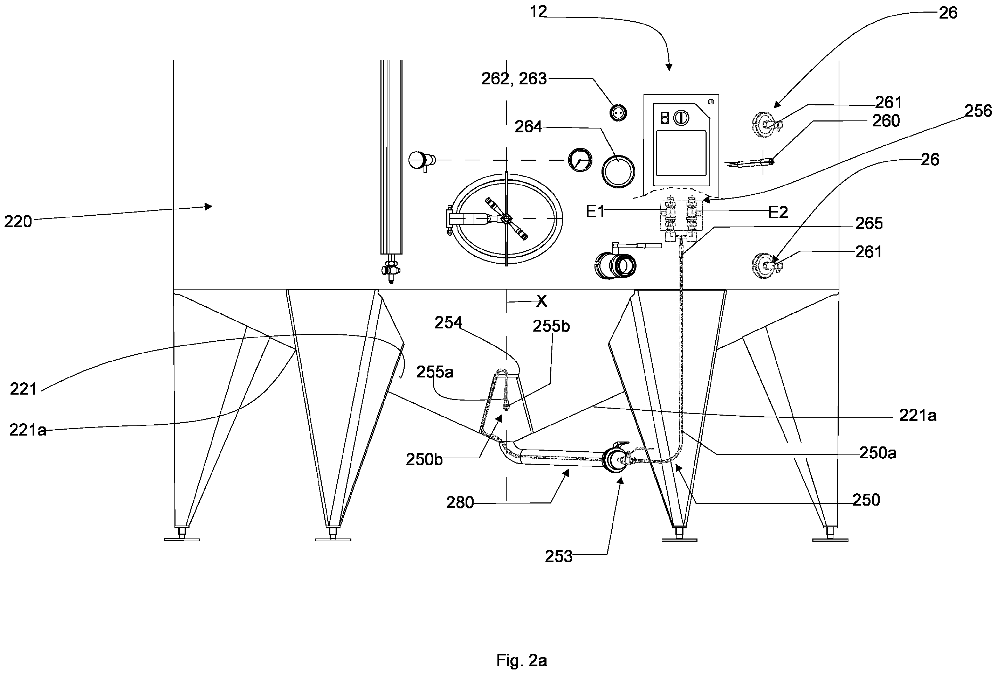

A preferred embodiment, depicted in Figures 1a, 2a, 3a, provides that said distributor 250b is shaped like a ball 255b.

In particular, said ball 250b is positioned substantially in the centre of tank 22 preferably near the bottom wall 221a.

In the event that the tank 22 has a conical bottom wall 221a, as depicted in the figures, said ball 255b is preferably positioned within the conical portion of the bottom 221.

In this case, the ball 250b is preferably positioned at the axis of symmetry X of the cone defined by the bottom wall 221a.

In particular, the portion of conduit 255a descends downwards so that it ends with the ball 255b facing the bottom wall 221a.

Said portion of the conduit 255a and the ball 255b can be supported by a special support stand 254 resting on the bottom wall 221a.

Said ball 255b preferably has a diameter comprised between 25 and 50mm.

The ball 255b has the advantage of having a conformation without recesses or loops, which are difficult to clean, therefore said solution has the advantage of being easy to wash and clean.

Said ball 255b preferably comprises a plurality of holes distributed on its outer surface as depicted in Figure 3a.

According to an embodiment not shown in the figure, said holes can only be distributed on the portion of the lower hemisphere facing the bottom wall 221a.

Preferably said holes have a diameter comprised between 1 and 3 mm, preferably 1.5 mm.

This measurement is a good compromise between the need to resuspend most of the lees deposited on the bottom 221 and to achieve homogeneous diffusion of the gas bubbles in the liquid.

In the case where the tank 22 has a large diameter, for example for tanks with a diameter of more than 3000 mm, a plurality of balls 255b can be provided, positioned inside the tank 22 and preferably at its bottom portion 220, in particular positioned near the bottom wall 221a.

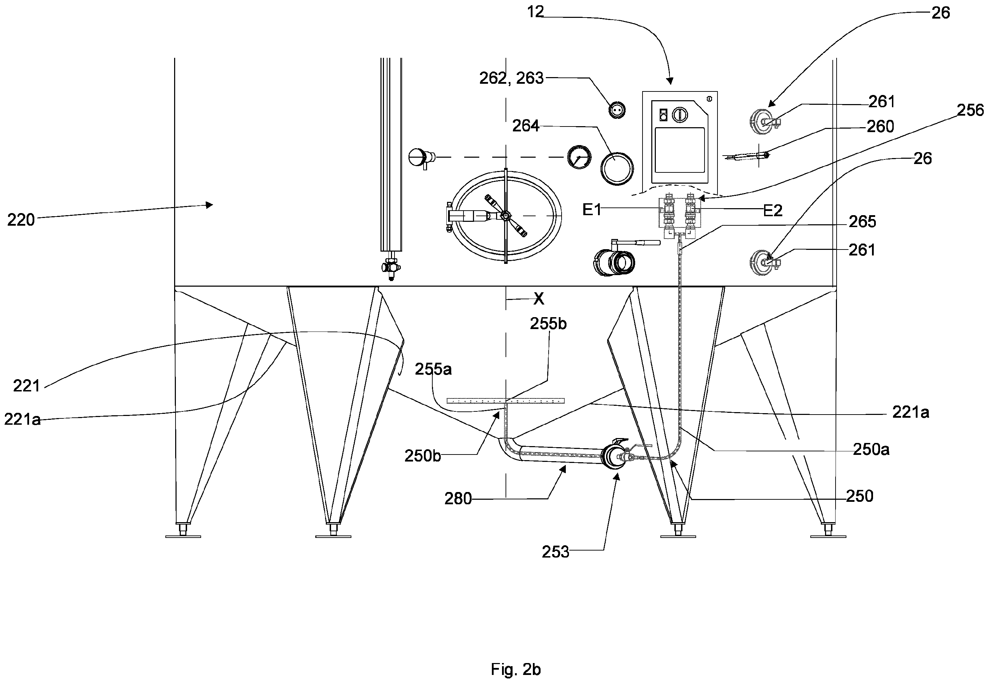

According to an alternative embodiment, depicted in Figures 1b, 2b, 3b, said distributor 250b is ring-shaped 255b.

Said ring 255b is preferably a tube bent into a ring and arranged on a horizontal plane.

In the event that the tank 22 has a conical bottom wall 221a, as depicted in the figures, said ring 255b is preferably positioned in the conical portion of the bottom 221.

Preferably, the axis of symmetry of the ring 255b coincides with the axis of symmetry X of the cone defined by the bottom wall 221a.

In particular, the portion of conduit 255a rises from the bottom wall 221a upwards to terminate with the ring 255b.

Said ring 255b preferably has a tube diameter comprised between 25 and 50mm and preferably the outer diameter of the ring is comprised between 250 and 500mm.

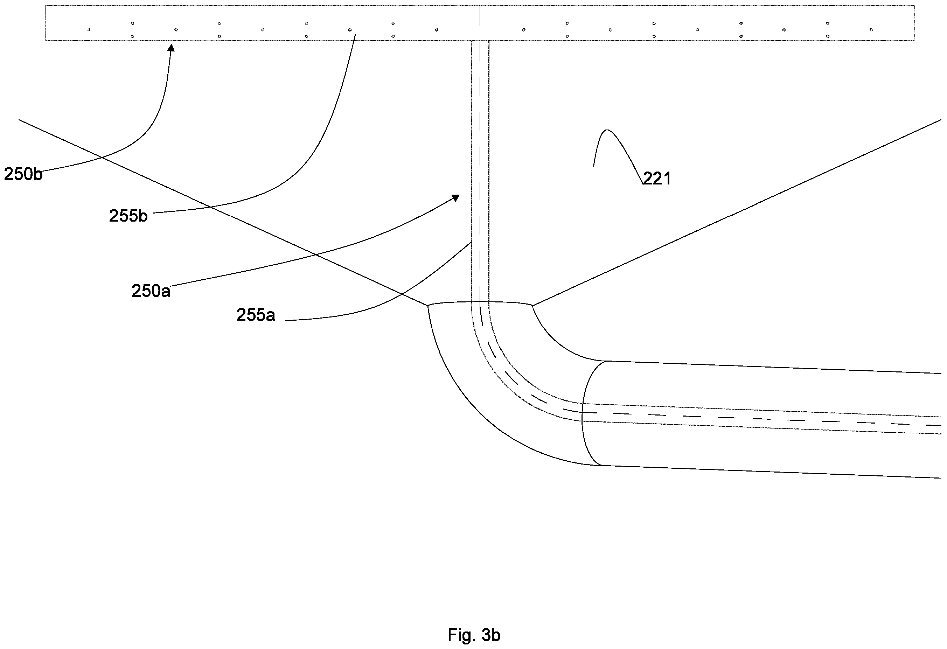

Said ring 255b preferably comprises a plurality of holes distributed on its outer surface as depicted in Figure 3b.

According to an embodiment, said holes can only be distributed on the portion of the lower ring facing the bottom wall 221a.

Preferably said holes have a diameter comprised between 1 and 3 mm in diameter, preferably 1.5 mm.

This measurement is a good compromise between the need to resuspend most of the lees deposited on the bottom 221 and to achieve homogeneous diffusion of the gas bubbles in the liquid.

In the case where the tank 22 has a large diameter, for example for tanks with a diameter of more than 3000 mm, a plurality of rings 255b can be provided, positioned inside the tank 22 and preferably at its bottom portion 220, in particular positioned near the bottom wall 221a.

According to a preferred embodiment, the active means 24 comprise activation means 256 for placing in fluid connection, in particular in an alternative manner, said means 250 for introducing a gas with the first supply means 251 or with the second supply means 252.

Thanks to this solution, it is possible to choose to insufflate the first or second technical gas G1, G2 into the tank 22 using the same introducing means 250, in particular the same distributor 250b.

This makes the solution simple and inexpensive.

In a preferred embodiment, said activation means 256 define the time or intensity and/or frequency of the first G1 and/or second G2 gas introduced.

Said activation means 256 as depicted in the figure may comprise a first solenoid valve E1 operatively connected to the first supply means 251 for controlling the first gas G1 and a second solenoid valve E2 operatively connected to the second supply means 252 for controlling the second gas G2.

Acting on said activation means 256, the distributor 250b can perform, for example, an introduction of the first or second gas G1, G2 with a time/intensity and frequency such as to be able to bring back into suspension the solid components deposited on the bottom 221 of the tank 22, for example fine lees, in order to facilitate the extraction of noble substances and promote their solubilisation, without resorting to mechanical handling and without the need to move the liquid mass externally.

Furthermore, by varying the time/intensity and frequency of the first or second technical gas G1, G2, it is possible to vinify the same must, obtaining wines with different sensory profiles.

Downstream of the activation means 256, in particular downstream of the first solenoid valve E1 and the second solenoid valve E2, the conduit 250a is the same for supplying both the first gas G1 and the second gas G2.

This makes the solution simple and inexpensive.

According to an advantageous aspect of the invention, the distributor 250b, can also be used at the end of the vinification and once the tank 22 has been emptied, to perform at least a partial flushing of said tank 22.

In such a case, the conduit 250a may comprise shut-off means 253, for example a quick coupling and/or a check valve, to place it in fluid connection with an additional source of fluid, different from the first or second supply means 251, 252.

In this case, said additional supply source is configured for introducing liquid or vapour.

Thanks to this solution, the consumption of washing water for cleaning the bottom of the tank 22 can be reduced.

Said apparatus 10 is thus also an advantageous solution in terms of sustainability and reduction of waste.

According to a preferred configuration, the apparatus 10 comprises means 26 for detecting physical and/or chemical parameters of said must/wine.

According to an aspect of the invention, said detection means 26 are configured for detecting the flow of liquid and/or gaseous fluids within the conduit 250a, especially via a corresponding flow sensor 265.

Thanks to said flow sensor 265 it is possible to detect the first and/or second technical gas G1, Gg2.

Said flow sensor 265 is preferably positioned downstream of the activation means 256.

Preferably said flow sensor 265 is configured to detect the gas flow G1, G2 against a change in temperature.

The flow sensor 265 detects, according to the calorimetric measuring principle, the flow velocity.

Advantageously, said means 26 for detecting the physical and/or chemical parameters of said must/wine, allow in particular:
- the detection of the redox potential, especially through a corresponding sensor 262,
- the detection of the temperature, especially through a corresponding sensor 260,
- the detection of the must density, especially by means of a corresponding sensor 261, i.e. a first and a second density sensor 261, 261, preferably placed at different height levels, in order to obtain more reliable density measurements, and from the signals of these to obtain the alcohol developed through an algorithm of interpolation of the density data detected thereby,
- the detection of the amount of oxygen dissolved in the must, especially through a corresponding sensor 263,
- the detection of the colour especially through a corresponding sensor 264.

For example, said detection means 26 are configured to detect the redox potential, especially via the corresponding sensor 262 which is installed on the tank 22 in particular at the side surface and preferably at the lower portion 220 of the tank 22.

Thanks to the activation means 256 and the detection means 26, it is possible to automate the introduction of the first or second technical gas G1, G2 into the tank 22.

This allows controlled and timely intervention throughout the vinification process, which benefits the quality of the wine by reducing the onset of defects.

Advantageously, to further automate the introduction of the gases G1, G2 throughout the entire must vinification process, the apparatus 10 comprises processor means 12.

According to a preferred form, the apparatus 10 comprises input means 14 of corresponding data and/or signals into said processor means 12, and output means 16 of corresponding data and/or signals originating from said processor means 12.

In particular said input means 14 of corresponding data and/or signals into said processor means 12 comprise a respective keyboard, or equivalent, for input of corresponding commands and/or said means 26 of detecting physical and/or chemical parameters of said must/wine.

In particular, said output means 16 of corresponding data and/or signals originating from said processor means 12 comprise a display screen of said processor means 12 and/or said active means 24 on the must/wine.

In particular, said processor means 12 are configured to activate, in particular automatically, one or more of said active means 24 on the must/wine or in response to a signal received from said means 26 for detecting physical and/or chemical parameters of said must/wine.

Advantageously, according to a preferred procedure, following a signal received from said density sensor 261, and preferably the determination of the corresponding alcohol level, if the alcohol level is lower than the desired alcohol level said processor means 12 are configured to increase the temperature of said must, activating the corresponding conditioning means 242, if, on the other hand, said alcohol level is higher than the desired alcohol level, said processor means 12 are configured to decrease the temperature of said must, activating the corresponding conditioning means 242.

Advantageously, as illustrated, said processor means 12 are configured to control the vinification process or procedure, in particular implemented by said apparatus 10, in particular whose steps and activities can advantageously be deduced from a corresponding graphical user interface 30.

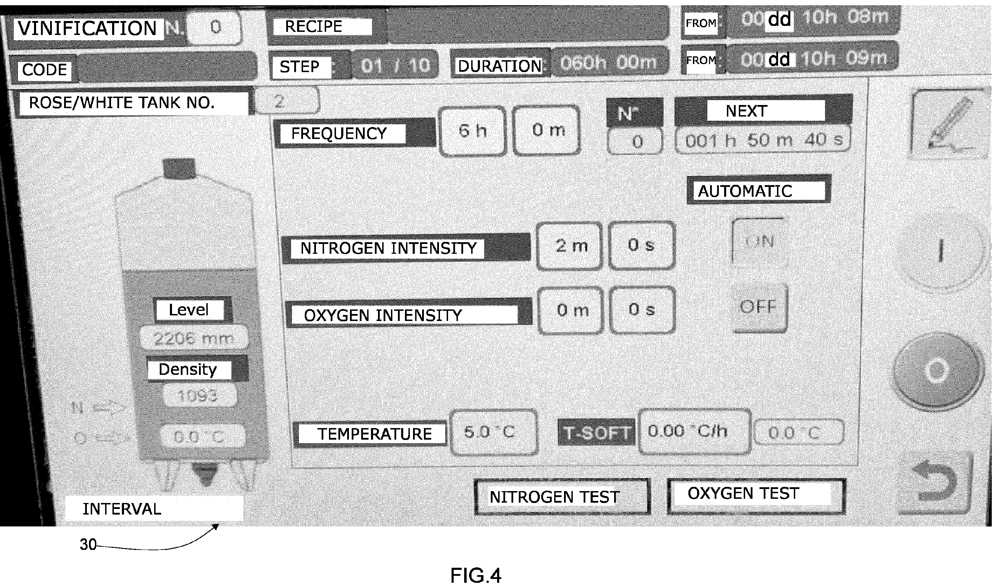

An example of a graphical user interface 30 is shown in Figure 4.

Advantageously, said processor means 12 are configured to display, in particular on a corresponding display 16, or on a remotely connected display, especially of said processor means 12, the alcohol trend in the vinification process, preferably which is generated from signals received by said density detection means 261 of said must.

In this way, it is possible to always know what stage of vinification is in progress and to constantly monitor the transition from the first settling step, to the second fermentation step, to the third refining step.

Consequently, it is possible to manage the introduction of the first or second technical gas G1 or G2 in a manner appropriate to the vinification underway.

By means of the processor means 12, it is possible to act on the activation means 256 to vary the time and/or frequency of introduction of the first gas G1 and/or second gas G2 in order to intervene on the various vinification steps by slowing them down or speeding them up.

According to a preferred embodiment, said processor means 12, following a signal received from the redox potential sensor 262, activate the first power supply means 251, if the detected potential is lower than the predetermined minimum potential.

Conversely, following a signal received from said redox potential sensor 262, if the potential detected is higher than the predetermined maximum potential, said processor means 12, block the first supply means 251.

Preferably said processor means 12 act on the activation means 256 to control the time and/or frequency of the first gas G1 and/or second gas G2 introduced.

Advantageously, according to a further preferred embodiment, following a further signal received from said dissolved oxygen sensor 263, if this dissolved oxygen value is not greater than a predetermined value, said processor means 12 are configured not to block aeration of said must, otherwise if this dissolved oxygen value is greater than a predetermined value, said processor means 12 are configured to block aeration of said must.

Said processor means 12 may advantageously use a recipe 181 or a collection or set 18 of vinification recipes 181, housed in a corresponding memory of, or in connection with, said processor means 12.

In this way, the vinification process can be optimally managed, avoiding or reducing the intervention of the operator or oenologist to a maximum degree, and thus minimising any errors.

Advantageously, said vinification recipes 181 can be displayed through said output means 16, in particular from the display of said processor means 12 on board said apparatus 10 for the vinification of corresponding must/wine, and yet could also be displayed on the display of any possibly remote processor means.

With appreciable advantage, according to another aspect of the process, said processor means 12 control the vinification apparatus 10, i.e. said means active 24 on the must/wine, to automatically execute the respective vinification recipe 181.

In this way, operator intervention in the vinification process can be partly or completely avoided and human errors and the costs resulting from them can be minimised, thus increasing the sustainability of the vinification process.

In an appreciably advantageous manner, according to another aspect of the procedure, it is provided that the vinification recipe 181 can be modified manually in progress, in particular by inputting corresponding data or signals into said processor means 12 of the apparatus, for example by means of the graphical user interface shown in Figure 12.

In this way, the operator can make his/her own specific contribution to the vinification process.

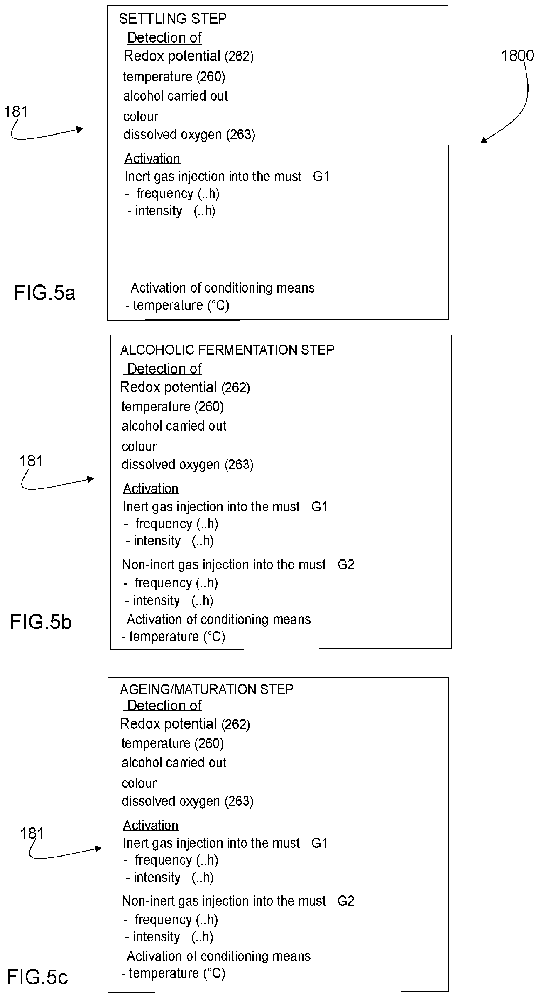

Advantageously, according to another aspect of the procedure, a new vinification recipe 181 can be envisaged to be introduced into the set of vinification recipes 18, in particular through said input means 14 of corresponding data and/or signals in said processor means 12, for example through the graphical user interface 1800 illustrated in Figure 5.

Advantageously, as can be deduced from the graphical user interface 30, 1800, said processor means 12 are configured to store the vinification process implemented and to allow it to be recalled by the operator. In particular, an advantageous recipe save button can be arranged in said interface 30, 1800. In this way, the process actually implemented can be the model for subsequent vinification procedures.

This process is then implemented, i.e. this apparatus is controlled, by a corresponding programme, software, or artificial intelligence, used by the processor means 12.

Advantageously, said processor means are, or comprise corresponding processor means which are remotely connected, for example by means of a communication network, in particular the global communication network, with said processor means 12 of said apparatus 10 for the vinification of corresponding must, and/or with said means active 24 on the must and/or said means 26 for detecting physical and/or chemical parameters of said must.

In this way, it is easy to operate remotely without necessarily having to be at the winemaker.

Further subject matter of the invention is the process that can be used for the vinification of a liquid must with its lees, in particular for white wine making, i.e. without marc, using the corresponding apparatus 10.

According to an aspect of the invention, said process involves introducing at least a first technical gas G1 into the must at a respective vinification stage and a second technical gas G2 of a different type at a respective vinification stage.

In particular, the introduction of said first technical gas G1 or second technical gas G2 takes place centrally at the tank 22.

According to an aspect of the invention, the process provides for carrying out, inside the tank 22, vinification steps, in particular for obtaining white or rosé wines, comprising a first settling step and/or a second alcoholic fermentation step and/or a third step of refining on the lees.

Advantageously, the process provides by means of the processor means 12 for activating the active means 24 in particular the first supply means 251 and/or the second supply means 252 in response to a signal received by the means 26 for detecting physical and/or chemical parameters of said must/wine.

Advantageously, according to a preferred embodiment, following a signal received from said redox potential sensor 262, if the detected potential is lower than the predetermined minimum potential, said processor means 12 are configured to activate the active means 24 in particular the supply means 252 to introduce the second technical gas G2 into the must by performing an aeration or oxygenation activity on the must.

Advantageously, according to a further preferred embodiment, following a signal received from said redox potential sensor 262, if the detected potential is higher than the predetermined maximum potential, said processor means 12 are configured to block the introduction of the second technical gas G2 to block aeration of said grape must.

According to an advantageous aspect of the invention said process provides by means of processor means 12 of the apparatus 10, to use at least one vinification recipe 181.

In particular, said recipe 181, or set 18 of vinification recipes 181, is housed in a corresponding memory of, or in connection with, said processor means 12.

In particular, said recipe 181 provides for the first gas G1 and/or the second gas G2 of different types to be introduced into the tank during the execution of a respective vinification step.

Advantageously, the process provides for the activation/deactivation of the active means 24 to be defined by the recipe 181.

Further preferably, the process provides that, by means of the recipe 181, the activation/deactivation time and/or their activation/deactivation frequency of introduction of the first gas G1 and the second gas G2 for each vinification step is defined.

According to an advantageous aspect, the process according to the invention may provide for activating the active means 24 either in response to a signal received by said detection means 26 of of physical and/or chemical parameters of said grape must, or according to times and quantities defined by a specific recipe 181 for the given type of wine to be obtained.

For example, it may be necessary to insufflate an inert gas G1 in a first settling step.

In this case, insufflating said inert gas G1 into the must is mainly intended to resuspend the solid material, lees and clarification substances.

This operation is generally activated according to a specific recipe 181.

Generally in this first step, the value detected by the redox potential sensor 262 is quite high, so it is generally not provided to introduce the second non-inert gas G2 in order not to activate oxidation on the must.

For example, in a second alcoholic fermentation step, it may be necessary to insufflate a non-inert G2 gas to provide the yeasts with the oxygen they need for better viability and utilisation of certain nitrogenous forms naturally present in the must, and to modulate the formation of fermentation aromas differently, thus obtaining different wines from the same must.

During this alcoholic fermentation step, it may also be necessary to insufflate an inert gas G1, if, for example, a reduction fermentation is to be carried out. This is typical of musts made from grapes with thiol precursors, or rich in norisoprenoids and/or terpenes.

The introduction of inert gas G1 or non-inert gas G2, in said second step, is generally triggered by the processor 12 according to the value detected by the redox potential sensor 262.

For example, in a third step of refining on the lees, it may be necessary to insufflate an inert gas G1 into the wine, the main purpose of which is to resuspend the solid material, i.e. the fermentation lees.

This operation is generally carried out according to a specific recipe 181.

In said third step of refining on the lees, it may also be necessary to insufflate a non-inert gas G2 such as air or oxygen, generally in low quantities. This non-inert gas makes it possible to avoid a state of excessive lees reduction when highly unpleasant sulphides would be formed and, therefore, detrimental to the quality of the wine, as precursors of mercaptans that are difficult to remove.

The introduction of non-inert gas G2 is generally triggered by the processor 12 according to the value detected by the redox potential sensor 262.

In practice, as is evident, the technical characteristics illustrated above allow, individually or in a respective combination, achieving one or more of the following advantageous results:
- it is possible to monitor and intervene promptly to guarantee the quality of the final product, in particular by dosing appropriately and selectively, technical gases, which are necessary for the correct performance of said vinification phases,
- long-term storage of the wine is possible, limiting oxidation to a minimum, and enhancing the structural component promoted by the release of noble substances from the lees placed in suspension,
- it is possible to vinify the same must to obtain wines with different sensory profiles.
- the vinification process can be optimally managed,
   avoiding or reducing to a maximum degree the intervention of the operator or oenologist,
- it is easy to operate remotely without necessarily having to be
   at the fermenter,
- the process actually implemented may be the basis
   for subsequent vinification processes.

The present invention is susceptible to evident industrial application. The person skilled in the art will also be able to imagine numerous modifications and/or variations to be made to the same invention, while remaining within the scope of the inventive concept, as extensively explained. Furthermore, the person skilled in the art will be able to imagine further preferred embodiments of the invention comprising one or more of the above illustrated features of the above-referenced preferred embodiment, in particular as set forth in the appended claims. Moreover, it must also be understood that all the details of the invention can be replaced by technically equivalent elements.

## Claims

1. Apparatus (10) usable for the vinification of a liquid must with its lees, in particular for white wine making, both of white grapes and of red grapes, said apparatus (10) comprising a tank (22) for containing the must/wine, **characterised in that** said tank (22) comprises active means (24) on the must/wine, in particular comprising means (250) for introducing a technical gas operatively connected to at least first supply means (251) for introducing into the must/wine at least a first technical gas (G1) and to at least second supply means (252) for introducing into the must/wine at least a second technical gas (G2) of a different type, wherein said means (250) for introducing a gas comprise a distributor (250b) placed within the tank (22) and positioned substantially in the centre of the tank (22).

2. Apparatus (10) according to any one of the preceding claims wherein the distributor (250b) is positioned at a lower portion (220) of the tank (22), in particular near a bottom wall (221a) thereof.

3. Apparatus (10) according to the preceding claim wherein the first technical gas (G1) is an inert gas and preferably comprises and/or consists of nitrogen and/or carbon dioxide.

4. Apparatus (10) according to any one of the preceding claims wherein the second technical gas (G2) is a non-inert gas and preferably comprises and/or consists of oxygen and/or air.

5. Apparatus (10) according to any one of the preceding claims wherein the active means (24) comprise activation means (256) for placing in fluid connection, in particular in an alternative manner, said means (250) of introducing a gas with the first supply means (251) or with the second supply means (252) and preferably for controlling the time and/or frequency of introducing the first gas (G1) and/or second gas (G2).

6. Apparatus (10) according to any one of the preceding claims wherein said means (250) for introducing a gas comprises a conduit (250a) in fluid connection with the distributor (250b), said conduit (250a) preferably being installed at a lower portion (220) of the tank (22) in particular near the bottom wall (221a) of the tank (22).

7. Apparatus (10) according to claim 6, wherein said conduit (250a) is connected to the tank (22) by entering from an outlet conduit (280) of said tank (22).

8. Apparatus (10) according to any one of the preceding claims wherein the distributor (250b) comprises a plurality of holes particularly distributed on its outer surface.

9. Apparatus (10) according to any one of the preceding claims wherein the distributor (250b) is shaped like a ball or ring (255b) preferably having a diameter comprised between 25 and 50 mm.

10. Apparatus (10) according to any one of the preceding claims wherein the distributor (250b), in particular the ball or ring (255b) comprises holes having a diameter comprised between 1 to 3 mm, preferably 1.5 mm.

11. Apparatus (10) according to any one of the preceding claims comprising means (26) for detecting physical and/or chemical parameters of said must/wine, in particular for detecting the temperature, especially by means of a corresponding sensor (260), for detecting the density of the must, especially by means of a corresponding sensor (261), i.e. a first and a second sensor (261, 261), for detecting the redox potential in particular by means of a corresponding sensor (262), for detecting the amount of oxygen dissolved in the must, in particular by means of a corresponding sensor (263), and detecting the colouring extracted during maceration, in particular by means of a corresponding sensor (264), and detecting the flow of liquid and/or gaseous fluids within the conduit (250a), in particular by means of a corresponding flow sensor (265).

12. Apparatus (10) according to any one of the preceding claims, comprising processor means (12), input means (14) of corresponding data and/or signals into said processor means (12), and output means (16) of corresponding data and/or signals originating from said processor means (12).

13. Apparatus (10) according to claim 12, **characterised in that** said input means (14) of corresponding data and/or signals into said processor means (12) comprise a respective keyboard, or equivalent, for input of corresponding commands and/or said means (26) of detecting physical and/or chemical parameters of said must/wine.

14. Apparatus (10) according to any one of claims 12 to 13, **characterised in that** said processor means (12), are configured to activate, in particular automatically, one or more of said active means (24) on the must/wine or in response to a signal received from said means (26) for detecting physical and/or chemical parameters of said must/wine.

15. A process usable for vinification of a must with its lees, in particular for white wine making, both of white grapes and of red grapes, using a corresponding apparatus (10) made according to the preceding claims, for vinification, said process **characterised in that** at least a first technical gas (G1) is introduced into the must/wine in a respective vinification step and a second gas (G2) of a different type at a respective vinification step, said introduction being made from the centre of the tank (22).
